(19) Europäisches Patentamt
European Patent Office
Office européen des brevets

(11) Publication number: **0 043 479**
**A1**

# (12) EUROPEAN PATENT APPLICATION

(21) Application number: **81104760.4**

(22) Date of filing: **14.12.79**

(51) Int. Cl.³: **C 07 C 49/753**, C 07 C 49/743, C 07 C 45/59, C 07 C 45/71 // C07D307/32

(30) Priority: **21.12.78 US 972005**

(43) Date of publication of application: **13.01.82 Bulletin 82/2**

(84) Designated Contracting States: **BE CH DE FR GB IT NL SE**

(60) Publication number of the earlier application in accordance with Art. 76 EPC: **0013107**

(71) Applicant: **AMERICAN CYANAMID COMPANY, Berdan Avenue, Wayne New Jersey 06904 (US)**

(72) Inventor: **Floyd, Middleton Brawner, Jr., 5 Babbling Bropok Lane, Suffern New York (US)**
Inventor: **Grudzinskas, Charles Vincent, 21 Fifth Avenue, Nyack New York (US)**
Inventor: **Weiss, Martin Joseph, 975 Phyllis Lane, Oradell New Jersey (US)**
Inventor: **Chen, Sow-Mei Lai, 7 Tulip Court, Park Ridge New Jersey (US)**

(74) Representative: **Allam, Peter Clerk et al, LLOYD WISE, TREGEAR & CO. Norman House 105-109 Strand, London WC2R 0AE (GB)**

## (54) Novel cyclopentenone derivatives and methods for the preparation of the novel compounds.

(57) Cyclopentenone compounds of formula I useful as intermediates in the preparation of certain substituted prostan-1-ols:

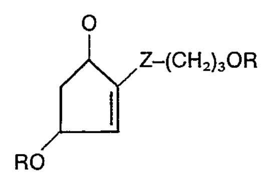

wherein Z is $-(CH_2)_4-$ or 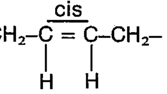

and both R groups are the same and are hydrogen or the group:

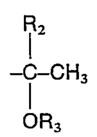

wherein $R_2$ is $(C_1-C_4)$ alkyl and $R_3$ is hydrogen or $(C_1-C_4)$ alkyl, with the proviso that Z is not $-(CH_2)_4-$ when R is hydrogen.

Processes for their preparation: by rearranging a substituted 2,5-dihydrofuran derivative of the formula:

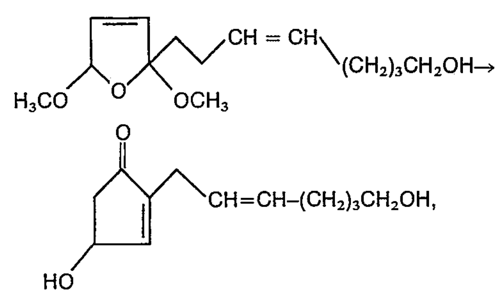

or by etherification of both OH functions in formula I with a 2-alkoxypropene of the formula

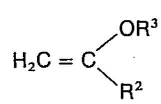

EP 0 043 479 A1

TITLE: NOVEL CYCLOPENTENONE DERIVATIVES AND METHODS FOR THE PREPARATION OF THE NOVEL COMPOUNDS

This invention relates to novel cyclopentenone derivatives which are useful intermediates in the preparation of certain of the 15-deoxy-16-hydroxy-16-vinyl (or cyclopropyl)-prostan-1-ols which form the subject of our European Application No. 79302907.5 from which the present application is divided.

The novel cyclopentenone compounds of the present invention may be represented by the formula:

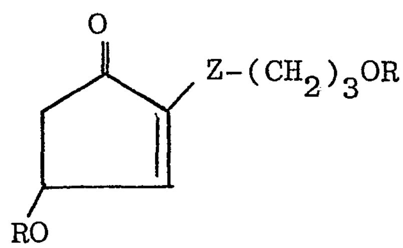

(I)

wherein Z is $-(CH_2)_4-$ or $-CH_2-\underset{H}{C}\overset{cis}{=}\underset{H}{C}-CH_2-$ and both R groups are the same and are hydrogen or the group:

$$-\underset{OR_3}{\overset{R_2}{C}}-CH_3$$

wherein $R_2$ is $(C_1-C_4)$ alkyl and $R_3$ is hydrogen or $(C_1-C_4)$alkyl; with the proviso that Z is not $-(CH_2)_4-$ when R is hydrogen.

The compound 2-(7'-hydroxy-2'-*cis*-heptenyl)-4-hydroxy-cyclopent-2-en-1-one, which is one of the compounds of this invention, may be prepared from 2-(7-carboxy-3-*cis*-heptenyl)-2,5-dimethoxy-2,5-dihydrofuran represented as II,

according to Flowchart A.

FLOWCHART A

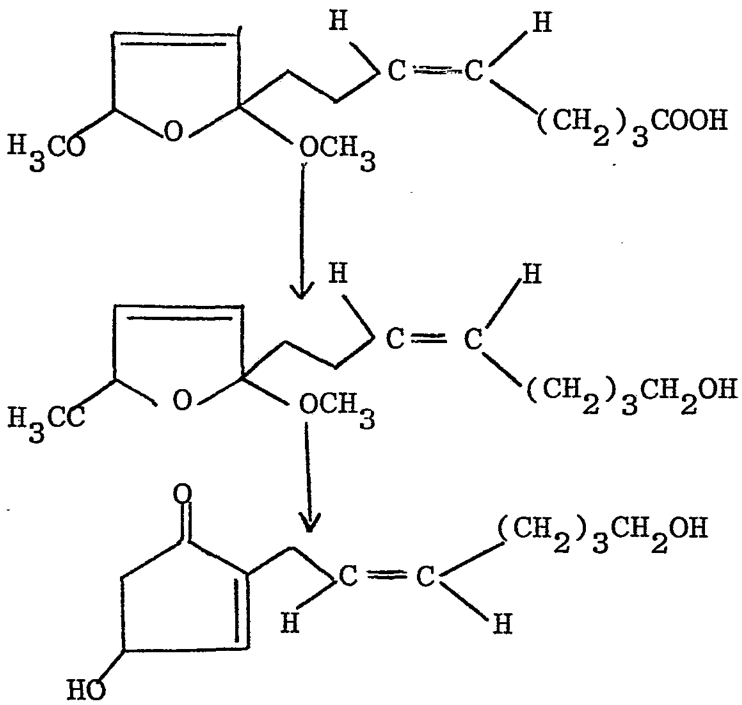

II

III

I

In accordance with the procedure outlined, the carboxyl group on the side chain of 2,5-dimethoxy furan is reduced to an alcohol by the use of sodium <u>bis</u>-(2-methoxyethoxy) aluminum hydride. Subsequently, the product (III) is rearranged to form the required 4-hydroxycyclopentenone. The saturated side chain cyclopentenone has been prepared and the procedure is reported by Kluender and Peruzzotti, Tetrahedron Letters, No. 24, 2063-2066 (1977).

Compounds of this invention of the formula:

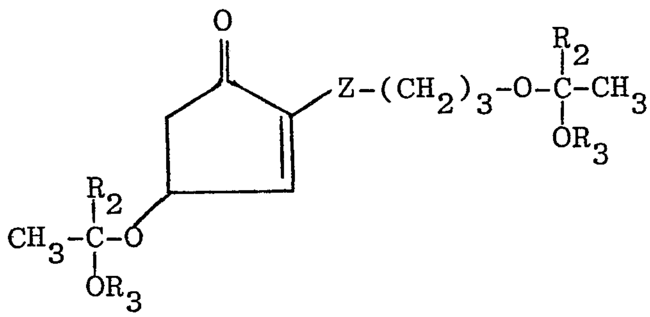

wherein Z, $R_2$ and $R_3$ are as defined above, may be prepared from 2-(7'-hydroxy-2'-cis-heptenyl)-4-hydroxy-cyclopent-2-en-1-one or from 2-(7'-hydroxy-heptyl)-4-hydroxy-cyclopent-2-en-1-one and a 2-alkoxypropene such as 2-methoxypropene or 2-ethoxyethylene according to Flowchart B.

FLOWCHART B

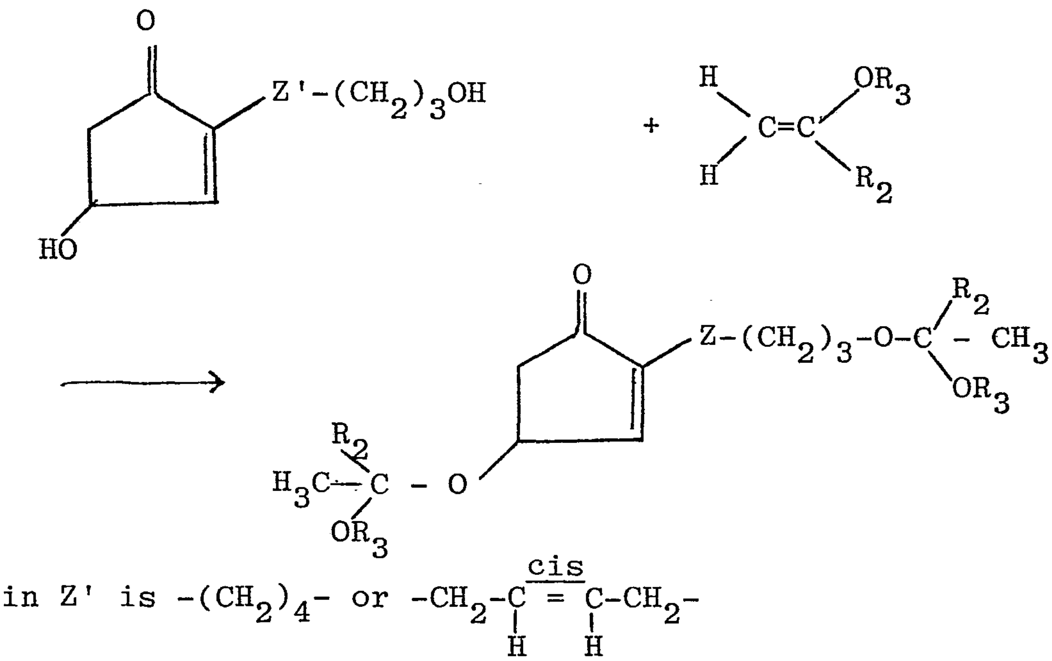

wherein Z' is $-(CH_2)_4-$ or $-CH_2-\underset{H}{C}\overset{cis}{=}\underset{H}{C}-CH_2-$

and $R_2$ and $R_3$ are as defined above.

According to Flowchart B above, the 2-alkoxypropene is added under pressure to a methylene chloride solution of the cyclopentenone at $10^{o}$-$15^{o}C$. A few drops of dichloroacetic acid is added to the mixture. The solution is stirred and alkoxyether is extracted into hexane.

The use of compounds of this invention in the preparation of 15-deoxy-16-hydroxy-16-vinyl (or cyclopropyl)-prostan-1-ols is shown in the description of the parent application.

The present invention is illustrated by the Examples which follow:

EXAMPLE 1

Preparation of 2,5-dihydro-2,5-dimethoxy-2-(8'-hydroxy-3'-cis-octenyl) furan

To a stirred solution of 10 ml of 3.6M sodium bis-(2-methoxyethoxy)aluminum hydride in toluene is slowly added a solution of 4.85 g of 2,5-dihydro-2,5-dimethoxy-2-(7'-carboxy-3'-cis-heptenyl) furan in 90 ml of toluene over an interval of 60 minutes, with the temperature maintained at 75°C. The mixture is then heated at 75°C for 2 hours, cooled to 0°C and diluted with 50 ml of ether. 20 ml of 20% aqueous sodium hydroxide is added dropwise to the mixture. The organic layer is separated, washed with saturated sodium chloride solution and dried over anhydrous potassium carbonate. The solvent is evaporated to provide the title compound.

EXAMPLE 2

Preparation of 2-(7'-hydroxy-2'-hydroxy-2'-cis-heptenyl)-4-hydroxy cyclopent-2-en-1-one

A stirred solution of 5.12 g of 2,5-dihydro-2,5-dimethoxy-2-(8'-hydroxy-3'-cis-octenyl)furan, 2.65 g of sodium dihydrogen phosphate monohydrate, 535 mg of anhydrous sodium acetate, and 20 mg of hydroquinone in 135 ml of dioxane and 68 ml of water is refluxed for 24 hours. The stirred solution is cooled to 50°C and

treated dropwise with 5.4 ml of concentrated sulfuric acid. The resulting solution is refluxed for 18 hours and then cooled to 25°C. Sodium chloride is added to form a saturated solution which is then extracted with ethyl acetate. The extract is washed with brine, dried over magnesium sulfate, and concentrated to afford the title compound.

EXAMPLE 3

Preparation of 4-1(1-methoxy-1-methylethoxy)-2-[7-(1-methoxy-1-methylmethoxyl)-heptyl]-cyclopent-2-en-1-one

To a solution of 3.57 g of 4-hydroxy-2-(7-hydroxyheptyl)-cyclopent-2-en-1-one in 27 ml of methylene chloride, under a positive pressure of argon at 10°-15°C, is added rapidly by pipet 5.4 ml of methoxypropene. 0.1 ml of dichloroacetic acid is then added dropwise and the solution stirred at ambient temperature for 4 hours and addition of a few more drops of dichloroacetic acid causes no reaction. The solution is diluted with 250 ml of hexane and shaken with 200 ml of saturated sodium bicarbonate solution. The layers are separated. The aqueous phase is extracted twice with 100 ml each of hexane. The combined organic layers are washed with 250 ml of saturated sodium chloride solution and dried over sodium sulfate. The salt is filtered off and the solution concentrated under reduced pressure. The product is a yellow oil weighing about 4.6 g and used without further purification.

EXAMPLE 4

In the manner of Example 3, the 4-hydroxy-2-(7'-hydroxy-2'-cis-heptenyl)cyclopent-2-en-1-one of Example 2 can be converted to its corresponding alkoxyether, 4-(1-methoxy-1-methylmethoxy)-2-[1-methoxy-1-methylmethoxy)-2-cis-heptenyl]-cyclopent-2-en-1-one.

EXAMPLES 5-6

In the manner of Example 3, the following 4-hydroxy-(7'-hydroxy)-cyclopentenones can be converted to their corresponding ethoxyethoxyethers by using ethylvinyl ether instead of methoxypropene.

| Example | Starting Cyclopentenone | Product 4-ethoxyethoxy-(7-ethoxy-ethoxy)cyclopent-2-en-1-one |
|---|---|---|
| 5 | 4-hydroxy-(7'-hydroxy-heptyl)-cyclopent-2-en-1-one | 4-ethoxyethoxy-(7'-ethoxy-ethoxypheptyl)-cyclopent-2-en-1-one |
| 6 | 4-hydroxy-(7'-hydroxy-2'-cis-heptenyl)-cyclopent-2-en-1-one | 4-ethoxyethoxy-(7'-ethoxy-ethoxy-2'-cis-heptenyl)-cyclopent-2-en-1-one |

CLAIMS:

1. A cyclopentenone compound of the general formula:

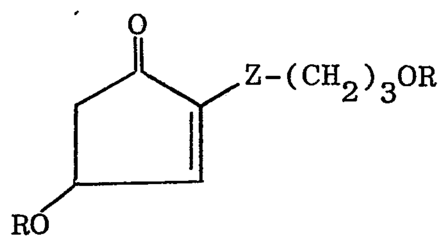

wherein Z is $-(CH_2)_4-$ or 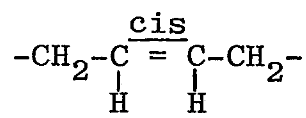

and both R groups are the same and are hydrogen or the group:

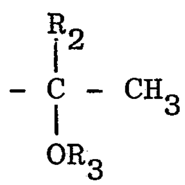

wherein $R_2$ is $(C_1-C_4)$alkyl and $R_3$ is hydrogen or $(C_1-C_4)$alkyl; with the proviso that Z is not $-(CH_2)_4-$ when R is hydrogen.

2. 2-(7'-Hydroxy-2'-cis-heptenyl)-4-hydroxy-cyclopent-2-en-1-one.

3. 4-(1-Methoxy-1-methylethoxy)-2-[7-(1-methoxy-1-methylethoxy)-2-cis-heptenyl]-cyclopent-2-en-1-one.

4. 4-(1-Ethoxy-1-ethoxy)-2[7-(1-ethoxy-1-ethoxy)-2-cis-heptenyl]-cyclopent-2-en-1-one.

5. A method of preparing a compound according to Claim 1 of the formula:

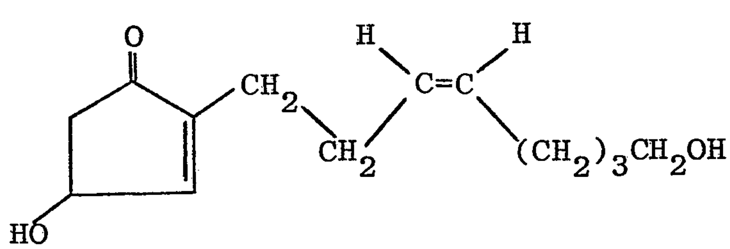

comprising the steps of:

(1) reducing 2,5-dihydro-2,5-dimethoxy-2-(7'-carboxy-3'-cis-heptenyl)-furan with sodium bis-(2-methoxyethoxy)aluminium hydride, to form 2,5-dihydro-2,5-dimethoxy-2-(8'-hydroxy-3'-cis-octenyl)furan, and thereafter

(2) rearranging the product of step (1) to form the desired 2-(7'-hydroxy-2'-cis-heptenyl)-4-hydroxy-cyclopent-2-en-1-one.

6. A method of preparing a compound according to Claim 1 of the formula:

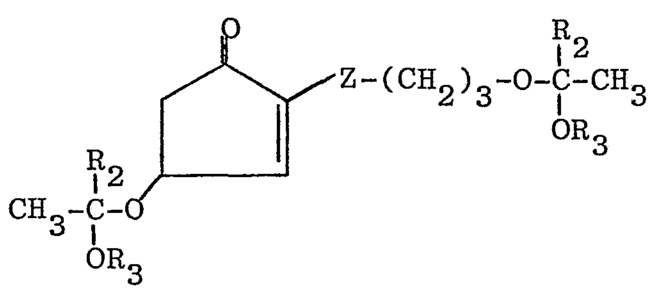

wherein Z, $R_2$ and $R_3$ are as defined in Claim 1, comprising reacting a compound of the formula:

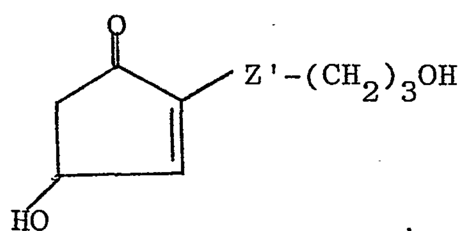

wherein Z' is $-(CH_2)_4-$ or $-CH_2-\underset{H}{C}\overset{cis}{=}\underset{H}{C}-CH_2-$, with a 2-alkoxypropene of the formula:

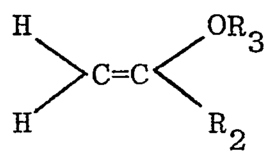

wherein $R_2$ and $R_3$ are as defined in Claim 1.

LLOYD WISE, TREGEAR & CO.
NORMAN HOUSE 105-109 STRAND
LONDON. WC2R 0AE

European Patent Office

# EUROPEAN SEARCH REPORT

0043479

Application number
EP 81 10 4760

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| D/X | TETRAHEDRON LETTERS, no. 24, 1977, Pergamon Press, GB<br>H.C. KLUENDER et al.: "Synthesis and Biological activity of some $PGE_1$ carbinols", pages 2063-2066<br>* Page 2064, compound 5a; page 2065, reaction scheme *<br>--- | 1,6 | C 07 C 49/753<br>49/743<br>45/59<br>45/71//<br>C 07 D 307/32 |
| X | US - A - 4 127 612 (H.C. KLUENDER et al.)<br>* Column 5, compound VI; column 13, lines 16-22 *<br>--- | 1,6 | |
| | | | TECHNICAL FIELDS SEARCHED (Int. Cl.3) |
| X | FR - A - 2 366 250 (MILES LABS)<br>* Claim 1; page 15, line 15 to page 16, line 1 *<br>& US - A - 4 074 063<br>& US - A - 4 108 892<br>& US - A - 4 115 438<br>& US - A - 4 115 453<br>& US - A - 4 149 017<br>--- | 1 | C 07 C 49/753<br>49/743<br>45/59<br>45/71 |
| P/X | GB - A - 2 009 162 (AMERICAN CYANAMID)<br>* Page 29, examples 155,153,156 *<br>--- | 1-6 | |
| | | | CATEGORY OF CITED DOCUMENTS |
| | CHEMICAL ABSTRACTS, vol. 86, 1977, page 491, abstract 89227a, COLUMBUS, OHIO (US)<br>T. SHONO et al.: "A facile and general synthesis of 4-hydroxy-cyclopentenones"<br>& Chem. Lett. 1976, (11), 1249-52<br>* The whole abstract *<br>-------- | 5 | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| The Hague | 24.08.1981 | BONNEVALLE |